# EUROPEAN PATENT APPLICATION

(11) **EP 4 520 757 A1**
(43) Date of publication of application: **12.03.2025**
(21) Application number: 23799653.3
(22) Date of filing: 02.05.2023
(51) Int. Cl.: C07D 405/14, A61K 31/506, A61P 35/00, A61P 31/12, A61P 25/16, A61P 1/16, A61P 31/06

(54) **COMPOUND INCLUDING 2,4-DIAMINOPYRIDINE, PREPARATION METHOD THEREFOR, AND PHARMACEUTICAL COMPOSITION CONTAINING SAME AS ACTIVE INGREDIENT FOR PREVENTION OR TREATMENT OF CANCER**

(30) Priority: 03.05.2022 KR 20220055086
(71) Applicant: Korea Research Institute of Chemical Technology, Daejeon 34114 (KR)
(72) Inventor: KIM, Pil Ho, Daejeon 34114 (KR); KIM, Seong Hwan, Daejeon 34114 (KR); KIM, Eun Hye, Daejeon 34114 (KR); AHN, Min Jeong, Daejeon 34114 (KR)
(74) Representative: Kraus & Lederer PartGmbB
(86) International application number: PCT/KR2023/005982
(87) International publication number: WO 2023/214772

(57) **Abstract**

The present disclosure relates to an isobenzofuran-1(3H)-one derivative, which is a kinase inhibitor, and a use thereof and, more specifically, to an isobenzofuran-1(3H)-one derivative having HPK1 inhibitory activity and MLK3 inhibitory activity and a pharmaceutical composition containing same for preventing or treating cancer, virus infectious diseases, Parkinson's disease, non-alcoholic steatohepatitis, or tuberculosis. In addition, the compound can be advantageously used as a composition for prevention or treatment of cancer as it is administered in combination with an anticancer agent or a cell therapy product.

## Description

### Technical Field

The present disclosure relates to an isobenzofuran-1(3H)-one derivative and uses thereof and, more specifically, to an isobenzofuran-1(3H)-one derivative with HPK1 inhibitory activity and MLK3 inhibitory activity, and a pharmaceutical composition containing same for the prevention or treatment of cancer, viral infectious diseases, Parkinson's disease, non-alcoholic steatohepatitis, or tuberculosis.

### Background Art

Protein kinases, which are enzymes that catalyze the transfer of the terminal phosphate group of adenosine triphosphate (ATP) to specific residues (tyrosine, serine, or threonine) on proteins, are involved in signals that regulate the activation, growth, and differentiation of cells in response to external mediators and environmental changes.

Generally, protein kinases are classified into serine/threonine kinase groups and tyrosine kinase groups depending on the substrates they phosphorylate (Hanks, S. K. et al., FASEB J., 9(8), 576-596, 1995). The serine/threonine kinase group includes protein kinase C isoforms (Newton, A. C., J Biol Chem., 270(48), 28495-28498, 1995), cyclin-dependent kinase groups, and cdc2 (Pines, J., Trends Biochem Sci., 18(6), 197, 1993). The tyrosine kinase group includes membrane-spanning growth factor receptors such as epidermal growth factor receptors (Iwashita, S. et al., Cell Signal., 4(2), 123-132, 1992), cytoplasmic non-receptor kinases such as p56tck, p59fYn, ZAP-70, and C-terminal Src kinases (Chan, A. C. et al., Annu Rev Immunol., 12, 555-592, 1994).

Inappropriately elevated protein kinase activity is directly or indirectly associated with numerous diseases resulting from abnormal cellular functions. For example, diseases can arise from failure in the proper regulatory mechanisms of kinases related to mutations, overexpression, or inappropriate enzyme activity, or from excess or deficiency in factors involved in signaling upstream or downstream of cytokines or kinases. Thus, the selective inhibition of kinase activity could serve as a valuable target for drug development for disease treatment.

MLK3, one of the mixed lineage kinase (MLK) family members, plays an important role in regulating MAP kinases, particularly JNK, which are critical for the proliferation and survival of cancer cells through various signaling pathways.

Additionally, there has been increasing research focused on developing inhibitors of MLK3 enzymatic activity for the treatment of cancer, viral infectious diseases, Parkinson's disease, non-alcoholic steatohepatitis, and other conditions (Chadee, D. N., Can J Physiol Pharmacol., 91(4), 268-274, 2013; Rattanasinchai, C. et al., Cancers (Basel)., 8(5), 51, 2016; Xu, H. et al., J Virol., 93(18), e00758-19, 2019; Saminathan, P. et al., J Neuroimmune Pharmacol., 14(1), 44-51, 2019; Goodfellow, V. S. et al., J Med Chem., 56(20), 8032-8048, 2013; Jiang, J. X. et al., Liver Int., 34(8), 1131-1132, 2014; Tomita, K. et al., JCI Insight., 2(15), 94488, 2017; Ibrahim, S. H. et al., Liver Int., 34(3), 427-437, 2014; Parkinson Study Group PRECEPT Investigators, Neurology, 69(15), 1480-1490, 2007; Kline, E. M. et al., Exp Neurol., 318, 157-164, 2019).

Hematopoietic progenitor kinase 1 (HPK1), initially cloned from hematopoietic progenitor cells, belongs to the MAP kinase kinase kinase kinase (MAP4K) family, which includes MAP4K1/HPK1, MAP4K2/GCK, MAP4K3/GLK, MAP4K4/HGK, MAP4K5/KHS, and MAP4K6/MINK (Hu, M.C., et al., Genes Dev, 1996. 10(18): p. 2251-64). HPK1 is predominantly expressed in hematopoietic cells such as T cells, B cells, macrophages, dendritic cells, neutrophils, and mast cells, making it of particular interest (Hu, M.C., et al., Genes Dev, 1996. 10(18): p. 2251-64; Kiefer, F., et al., EMBO J, 1996. 15(24): p. 7013-25). HPK1 kinase activity is induced by the activation of T cell receptors (TCR) (Liou, J., et al., Immunity, 2000. 12(4): p. 399-408), B cell receptors (BCR) (Liou, J., et al., Immunity, 2000. 12(4): p. 399-408), transforming growth factor receptors (TGF-βR) (Wang, W., et al., J Biol Chem, 1997. 272(36): p. 22771-5; Zhou, G., et al., J Biol Chem, 1999. 274(19): p. 13133-8), or Gs-coupled PGE2 receptors (EP2 and EP4) (Ikegami, R., et al., J Immunol, 2001. 166(7): p. 4689-96). Thus, HPK1 regulates various functions of immune cells.

HPK1 plays a significant role in the regulation of immune cell functions and has been implicated in autoimmune diseases and anti-tumor immunity (Shui, J.W., et al., Nat Immunol, 2007. 8(1): p. 84-91; Wang, X., et al., J Biol Chem, 2012. 287(14): p. 11037-48). HPK1 knockout mice were found to be more susceptible to the induction of experimental autoimmune encephalomyelitis (EAE) (Shui, J.W., et al., Nat Immunol, 2007. 8(1): p. 84-91). In humans, HPK1 was downregulated in peripheral blood mononuclear cells of patients with psoriatic arthritis and in T cells of patients with systemic lupus erythematosus (SLE) (Batliwalla, F.M., et al., Mol Med, 2005. 11(1-12): p. 21-9). These observations suggest that the reduction in HPK1 activity may contribute to the autoimmune nature of the disease. Furthermore, HPK1 can regulate anti-tumor immunity via T cell-dependent mechanisms. In a PGE2-producing lung carcinoma tumor model, tumors developed more slowly in HPK1 knockout mice than in wild-type mice (see US 2007/0087988). Additionally, adoptive transfer of HPK1-deficient T cells was shown to be more effective in controlling tumor growth and metastasis compared to wild-type T cells (Alzabin, S., et al., Cancer Immunol Immunother, 2010. 59(3): p. 419-29). Similarly, bone marrow-derived dendritic cells (BMDCs) from HPK1 knockout mice were more effective in enhancing T cell responses to eliminate Lewis lung carcinoma compared to wild-type BMDCs (Alzabin, S., et al., J Immunol, 2009. 182(10): p. 6187-94). Additionally, high HPK1 expression in cancer patients was reported to be associated with lower survival rates compared to patients with low HPK1 expression. Suppressing HPK1 expression in cancer cells inhibited tumor growth, and inhibiting HPK1 expression in various CAR-T cells, such as CD19 CAR-T, Her2 CAR-T, and BCMA (B cell maturation antigen) CAR-T, enhanced the anti-tumor immune capacity of CAR-T cells, effectively suppressing cancer cell proliferation and growth (Si, J., et al., Cancer Cell, 2020, 38(4): p. 551-66). These data, together with the limited expression of HPK1 in hematopoietic cells and the absence of any adverse effects on immune cell development, suggest that HPK1 could be an excellent drug target for enhancing anti-tumor immunity. Additionally, in immunotherapy using T cells, ex vivo cultivation is crucial, and technologies that enable the rapid proliferation of large numbers of high-quality T cells in culture are necessary (Dudley, ME., et al., Nat. Rev. Cancer, 2003, 3(9): p. 666-75). A new T cell culture technology for immunotherapy can be developed by combining techniques that regulate hematopoietic cell differentiation and proliferation within culture vessels. Thus, there is a need for novel compounds that regulate HPK1 activity.

In prior art related to isobenzofuran-1(3H)-one derivatives that exhibit HPK1 inhibitory activity and MLK3 inhibitory activity, as well as pharmaceutical compositions containing the same for the prevention or treatment of cancer, viral infectious diseases, Parkinson's disease, non-alcoholic fatty liver disease, or tuberculosis, Korean Patent No. 10-0832602 discloses novel polycyclic compounds associated with the activity of MLK3 enzymes and their uses. However, there has been no prior report mentioning isobenzofuran-1(3H)-one derivatives, such as those represented by Chemical Formulae 1 and 2 of the present disclosure, along with their HPK1 and MLK3 inhibitory activities and their potential for treating cancer, viral infectious diseases, Parkinson's disease, non-alcoholic fatty liver disease, or tuberculosis.

Accordingly, during the research process on isobenzofuran-1(3H)-one derivatives, the present inventors confirmed both HPK1 inhibitory activity and MLK3 inhibitory activity, thereby completing the present disclosure.

### Disclosure of Invention

### Technical Problem

The present disclosure aims to provide isobenzofuran-1(3H)-one derivatives and uses thereof and, more specifically, to provide isobenzofuran-1 (3H) - one derivatives with HPK1 inhibitory activity and MLK3 inhibitory activity, and a pharmaceutical composition containing same for the prevention or treatment of cancer, viral infectious diseases, Parkinson's disease, non-alcoholic steatohepatitis, or tuberculosis.

### Solution to Problem

The present disclosure relates to a compound represented by Chemical Formula 1, 2, 3, or 4, or a pharmaceutically acceptable salt thereof.

The present disclosure also relates to a compound represented by Chemical Formula 1 or Chemical Formula 2, an optical isomer thereof, or a pharmaceutically acceptable salt thereof.

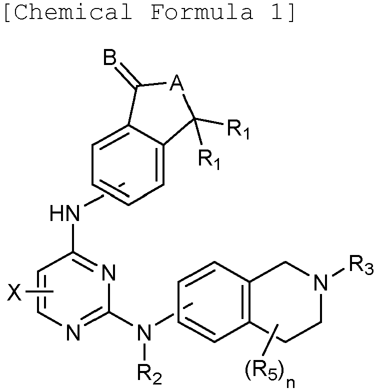

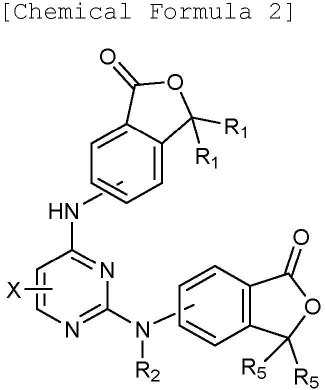

wherein,
R₁'s are each independently a hydrogen atom, a C₁-C₄ alkyl, or a C₁-C₄ alkoxy,
or the two R₁'s form a 3- to 7-membered saturated spiro ring with an adjacent carbon atom (C);
R₂ is a hydrogen atom, a C₁-C₄ alkyl, or a C₁-C₄ alkoxy;
R₃ is a hydrogen atom, a C₁-C₄ alkyl, a halo C₁-C₄ alkyl, a hydroxy C₁-C₄ alkyl, a C₁-C₄ alkoxy, a halo C₁-C₄ alkoxy, a C₁-C₄ alkoxy C₁-C₄ alkoxy, -COR₄, -CONH₂, - CONHR₄, -CON(R₄)₂, -SO₃H, or -SO₂R₄;
R₄ is a substituted or unsubstituted trigonal to dodecagonal cycloalkyl, a substituted or unsubstituted trigonal to dodecagonal heterocycloalkyl, a substituted or unsubstituted tetragonal to dodecagonal aryl, a substituted or unsubstituted tetragonal to dodecagonal heteroaryl, a substituted or unsubstituted trigonal to dodecagonal cycloalkyl C₁-C₄ alkyl, a substituted or unsubstituted trigonal to dodecagonal heterocycloalkyl C₁-C₄ alkyl, a substituted or unsubstituted tetragonal to dodecagonal aryl C₁-C₄ alkyl, a substituted or unsubstituted tetragonal to dodecagonal heteroaryl C₁-C₄ alkyl, a substituted or unsubstituted C₁-C₄ alkyl, a halo C₁-C₄ alkyl, a hydroxy C₁-C₄ alkyl, a C₁-C₄ alkoxy, a halo C₁-C₄ alkoxy, or a C₁-C₄ alkoxy C₁-C₄ alkoxy,
   wherein the substituted cycloalkyl, heterocycloalkyl, aryl, heteroaryl, cycloalkylalkyl, heterocycloalkylalkyl, arylalkyl, heteroarylalkyl, or alkyl has at least one substituent selected from the group consisting of OH, NO₂, NH₂, CN, a halogen, a C₁-C₄ alkyl, a halo C₁-C₄ alkyl, a hydroxy C₁-C₄ alkyl, a C₁-C₄ alkoxy, a halo C₁-C₄ alkoxy, and a C₁-C₄ alkoxy C₁-C₄ alkoxy, at a position to which a hydrogen atom can be bonded;
R₅'s are each independently a hydrogen atom, a C₁-C₄ alkyl, or a C₁-C₄ alkoxy,
or the two R₅'s form a 3- to 7-membered saturated spiro ring with an adjacent carbon atom (C);
A is O or NH;
B is O or S;
X is a halogen atom; and
n is an integer of 0 to 6.

Also, the present disclosure relates to a compound represented by the following Chemical Formula 3 or 4, an optical isomer thereof, or a pharmaceutically acceptable salt thereof. 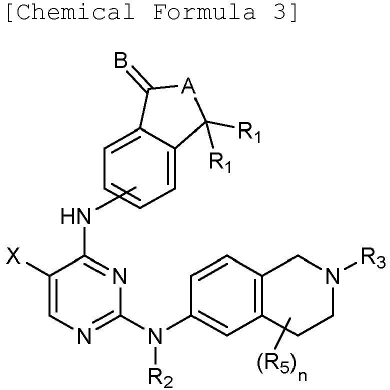 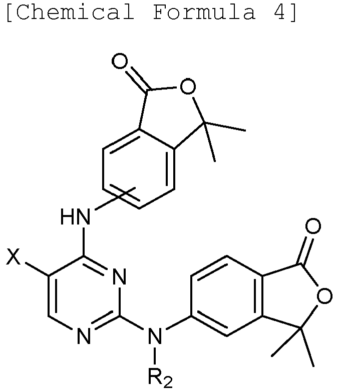 wherein,
R₁'s are each independently a hydrogen atom, a C₁-C₄ alkyl, or a C₁-C₄ alkoxy,
or the two R₁'s form a 3- to 7-membered saturated spiro ring with an adjacent carbon atom (C);
R₂ is a hydrogen atom, a C₁-C₄ alkyl, or a C₁-C₄ alkoxy;
R₃ is a hydrogen atom, a C₁-C₄ alkyl, a halo C₁-C₄ alkyl, a hydroxy C₁-C₄ alkyl, a C₁-C₄ alkoxy, a halo C₁-C₄ alkoxy, a C₁-C₄ alkoxy C₁-C₄ alkoxy, -COR₄, -CONH₂, - CONHR₄, -CON(R₄)₂, -SO₃H, or -SO₂R₄;
R₄ is a substituted or unsubstituted trigonal to dodecagonal cycloalkyl, a substituted or unsubstituted trigonal to dodecagonal heterocycloalkyl, a substituted or unsubstituted tetragonal to dodecagonal aryl, a substituted or unsubstituted tetragonal to dodecagonal heteroaryl, a substituted or unsubstituted trigonal to dodecagonal cycloalkyl C₁-C₄ alkyl, a substituted or unsubstituted trigonal to dodecagonal heterocycloalkyl C₁-C₄ alkyl, a substituted or unsubstituted tetragonal to dodecagonal aryl C₁-C₄ alkyl, a substituted or unsubstituted tetragonal to dodecagonal heteroaryl C₁-C₄ alkyl, a substituted or unsubstituted C₁-C₄ alkyl, a halo C₁-C₄ alkyl, a hydroxy C₁-C₄ alkyl, a C₁-C₄ alkoxy, a halo C₁-C₄ alkoxy, or a C₁-C₄ alkoxy C₁-C₄ alkoxy,
   wherein the substituted cycloalkyl, heterocycloalkyl, aryl, heteroaryl, cycloalkylalkyl, heterocycloalkylalkyl, arylalkyl, heteroarylalkyl or alkyl has at least one substituent selected from the group consisting of OH, NO₂, NH₂, CN, halogen, a C₁-C₄ alkyl, a halo C₁-C₄ alkyl, a hydroxy C₁-C₄ alkyl, a C₁-C₄ alkoxy, a halo C₁-C₄ alkoxy, and a C₁-C₄ alkoxy C₁-C₄ alkoxy, at a position to which a hydrogen atom can be bonded;
R₅'s are each independently a hydrogen atom, a C₁-C₄ alkyl, or a C₁-C₄ alkoxy,
or the two R₅'s form a 3- to 7-membered saturated spiro ring with an adjacent carbon atom (C);
A is O or NH;
B is O or S;
X is a halogen atom; and
n is an integer of 0 to 6.

Specifically, the compound is a compound represented by Chemical Formula 1, 2, 3, or 4, selected from the group consisting of: 5-((5-chloro-2-((1,2,3,4-tetrahydroisoquinolin-6-yl)amino)pyrimidin-4-yl)amino)-3,3-dimethylisobenzofuran-1(3H)-one (compound 1);
6-((5-chloro-2-((1,2,3,4-tetrahydroisoquinolin-6-yl)amino)pyrimidin-4-yl)amino)-3,3-dimethylisobenzofuran-1(3H)-one (compound 2); and
5,5'-((5-chloropyrimidine-2,4-diyl)bis(azanediyl))bis(3,3-dimethylisobenzofuran-1(3H)-one) (compound 3);
an optical isomer thereof, or a pharmaceutically acceptable salt thereof.

The terms used in the present disclosure have the following meanings unless otherwise indicated. Undefined terms should be understood as having their commonly accepted meaning in the relevant field.

As used herein, the term "halo" or "halogen" is used in its usual meaning to refer to a fluoro, chloro, bromo, or iodo substituent.

As used herein, the term "alkyl" refers to a hydrocarbon group, either straight or branched, with a single bond. Examples include methyl, ethyl, propyl, n-butyl, isobutyl, tert-butyl, 1-methylpropyl, among others.

As used herein, the term "alkoxy" refers to an oxygen radical bonded to a saturated hydrocarbon chain, which can be either straight or branched. Examples include methoxy, ethoxy, propoxy, n-butoxy, tert-butoxy, and 1-methylpropoxy.

As used herein, the term "haloalkyl" refers to an alkyl radical in which one or more hydrogen atoms have been replaced by halogen atoms, where "alkyl" and "halo" are as previously defined.

As used herein, the term "haloalkoxy" refers to an alkoxy radical in which one or more hydrogen atoms have been replaced by halogen atoms, with "halo" and "alkoxy" as defined above.

As used herein, the term "alkoxyalkoxy" refers to an alkoxy radical in which one or more hydrogen atoms are replaced by another alkoxy group, with "alkoxy" as defined above.

As used herein, the term "cyclic" or "ring" refers to a substituted or unsubstituted, mono-, bi-, or polycyclic aliphatic or aromatic radical, which may or may not bear a heteroatom.

As used herein, the term "cycloalkyl" refers to a saturated cyclic hydrocarbon radical. Examples include cyclopropyl, cyclobutyl, cyclopentyl, cyclohexyl, cycloheptyl, cyclooctyl, bicyclo[3.1.1]heptyl, spiro[4.5]decyl, spiro[5.5]undecyl, and adamantyl.

As used herein, the term "heterocycloalkyl" refers to a cyclic saturated hydrocarbon radical bearing one or more heteroatoms such as nitrogen (N), oxygen (O), or sulfur (S). Depending on the number and type of heteroatoms and the number of carbon atoms in the ring, examples include aziridinyl, pyrrolidinyl, piperidinyl, oxopiperidinyl, morpholinyl, piperazinyl, oxopiperazinyl, morpholinyl, thiomorpholinyl, azepanyl, diazepanyl, oxazepanyl, thiazepanyl, dioxothiazepanyl, azocanyl, tetrahydroisoquinolinyl, phthalanil, phthalanoyl, tetrahydrofuranil, tetrahydropyranyl, oxazolidinyl, dioxanyl, dioxolanil, etc.

As used herein, the term "aryl" refers to an aromatic substituent containing at least one ring with a conjugated π-electron system. Examples include phenyl, naphthyl, anthryl, biphenyl, and triphenyl.

As used herein, the term "heteroaryl" refers to an aromatic ring compound bearing one or more heteroatoms such as nitrogen (N), oxygen (O), or sulfur (S). According to the number and type of the heteroatoms contained in the ring and the number of carbon atoms, the heteroaryl may be exemplified by pyridyl, pyrrolyl, pyrrolidinyl, pyrimidinyl, furanyl, quinolizinyl, indolyl, pyrimidinyl, imidazolyl, 1,2,4-triazolyl, tetrazolyl, pyranyl, thiophenyl, thiazolyl, dibenzothiophenyl, dibenzofuranyl, dibenzoselenophene, thiophene, benzofuran, benzothiophenyl, benzoselenophenyl, carbazolyl, indolocarbazolyl, pyridylindolyl, pyrrolodipyridinyl, pyrazolyl, imidazolyl, triazolyl, oxazolyl, thiazolyl, oxadiazolyl, oxatriazolyl, dioxazolyl, thiadiazolyl, pyridinyl, pyridazinyl, pyrazinyl, triazinyl, oxazinyl, oxathiazinyl, oxadiazinyl, indolyl, benzimidazolyl, indazolyl, indoxazinyl, benzoxazolyl, benzisoxazolyl, benzothiazolyl, quinolinyl, isoquinolinyl, cinnolinyl, quinazolinyl, quinoxalinyl, naphthyridinyl, phthalazinyl, pteridinyl, xanthenyl, acridinyl, phenazyl, phenothiazinyl, phenoxazinyl, benzofuropyrimidinyl, furodipyrimidinyl, benzothienopyrimidinyl, thienodipyrimidinyl, benzoselenophenopyrimidinyl, selenophenodipyridine, and others.

As used herein, the term "cycloalkylalkyl" refers to a cycloalkyl radical substituted with an alkyl, with cycloalkyl and alkyl as previously defined.

As used herein, the term "heterocycloalkylalkyl" refers to a heterocycloalkyl radical substituted with an alkyl, with heterocycloalkyl and alkyl as previously defined.

As used herein, the term "arylalkyl" refers to an aryl radical substituted with an alkyl, with aryl and alkyl as defined above.

The term "heteroarylalkyl" refers to a heteroaryl group substituted with an alkyl group, with heteroaryl and alkyl as defined above.

Furthermore, the compounds of the present disclosure may contain one or more asymmetric carbon atoms and may exist as racemic mixtures or optically active forms. All such compounds, including their stereoisomers, fall within the scope of the present disclosure.

The term "pharmaceutically acceptable salt", as used herein, refers to a salt or complex of a compound represented by Chemical Formulas 1, 2, 3, or 4 that retains the desired biological activity. Examples of such salts include, but are not limited to, acid addition salts formed from inorganic acids (e.g., hydrochloric acid, hydrobromic acid, sulfuric acid, phosphoric acid, nitric acid, etc.) and salts formed from organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalenesulfonic acid, naphthalenedisulfonic acid, and polygalacturonic acid. The compound may also be administered as a pharmaceutically acceptable quaternary salt known to those skilled in the art, including, but not limited to, chloride, bromide, iodide, -O-alkyl, toluenesulfonate, methylsulfonate, sulfonate, phosphate, or carboxylate (e.g., benzoate, succinate, acetate, glycolate, maleate, malate, fumarate, citrate, tartrate, ascorbate, cinnamate, mandelate, and diphenylacetate). The compounds represented by Chemical Formulas 1, 2, 3, or 4 of the present disclosure may include pharmaceutically acceptable salts, as well as any salts, hydrates, solvates, and prodrugs prepared by conventional methods.

The acid addition salts of the present disclosure may be prepared by conventional methods, for example, by dissolving a derivative of a compound represented by Chemical Formulas 1, 2, 3, or 4 in an organic solvent such as methanol, ethanol, acetone, dichloromethane, or acetonitrile, adding an organic or inorganic acid, filtering the resulting precipitate, and then drying same. Alternatively, after adding an excess of acid, the solvent and acid are evaporated under reduced pressure and the residue is recrystallized in an organic solvent to afford the acid addition salts.

Furthermore, a pharmaceutically acceptable metal salt may be formed using a base. Alkali metal or alkaline earth metal salts may be prepared by, for example, dissolving the compound in an excess of an alkali metal hydroxide or alkaline earth metal hydroxide solution, filtering out the undissolved salt, and evaporating and drying the filtrate. Suitable metal salts for pharmaceutical use include sodium, potassium, or calcium salts. Additionally, the corresponding salts can be obtained by reacting the alkali metal or alkaline earth metal salts with appropriate silver salts (e.g., silver nitrate) .

Another aspect of the present disclosure pertains to a pharmaceutical composition for the prevention or treatment of cancer, viral infections, Parkinson's disease, non-alcoholic steatohepatitis (NASH), or tuberculosis, the composition containing a compound represented by Chemical Formulas 1, 2, 3, or 4, or an optical isomer or pharmaceutically acceptable salt thereof as an active ingredient. The compounds represented by Chemical Formulas 1, 2, 3, or 4 exhibit inhibitory activity against both HPK1 and MLK3.

The cancer may be selected from the group consisting of lung cancer, liver cancer, gastric cancer, colorectal cancer, bladder cancer, prostate cancer, breast cancer, ovarian cancer, cervical cancer, thyroid cancer, melanoma, hematologic cancers, colon cancer, non-small cell lung cancer, pancreatic cancer, skin cancer, head and neck cancer, small intestine cancer, rectal cancer, endometrial cancer, vaginal cancer, testicular cancer, esophageal cancer, bile duct cancer, lymphoma, gallbladder cancer, endocrine gland cancer, adrenal cancer, lymphoma, multiple myeloma, thymoma, mesothelioma, kidney cancer, brain cancer, central nervous system tumors, brainstem glioma, and pituitary adenoma, but with no limitations thereto.

The viral infectious disease may be infection caused by at least one virus selected from the group consisting of Zika virus, human immunodeficiency virus (HIV), influenza virus, Influenza A virus subtype H1N1, avian influenza virus, rhinovirus, adenovirus, coronavirus, parainfluenza virus, respiratory syncytial virus, herpesvirus (HSV), rotavirus, and hepatitis viruses, but with no limitations thereto.

Additionally, the present disclosure relates to a method for inhibiting kinase activity in a subject in need of inhibition of activity of kinases including HPK1, the method comprising administering an effective amount of a compound represented by Chemical Formulas 1, 2, 3, or 4, or a pharmaceutically acceptable salt or stereoisomer thereof, to the subject.

Furthermore, the present disclosure relates to a pharmaceutical composition for the prevention or treatment of cancer, characterized by the combined administration of a compound represented by Chemical Formulas 1, 2, 3, or 4, along with an anti-cancer drug.

The anti-cancer drug that may be co-administered may be selected from taxane-based anti-cancer drugs, antitumor alkylating agents, antitumor antimetabolites, antitumor antibiotics, plant-derived antitumor agents, antitumor platinum complexes, antitumor camptothecin derivatives, antitumor kinase inhibitors, antitumor antibodies, hormonal antitumor agents, antitumor viral agents, and angiogenesis inhibitors.

The taxane-based anti-cancer drugs may include paclitaxel, docetaxel, or cabazitaxel.

The antitumor alkylating agents may include nitrogen mustard N-oxide, cyclophosphamide, ifosfamide, melphalan, busulfan, mitobronitol, carboquone, thiotepa, ranimustine, nimustine, temozolomide, or carmustine.

The antitumor antimetabolites may include methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, or pemetrexed disodium.

The antitumor antibiotics may include actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, or valrubicin.

The plant-derived antitumor agents may include vincristine, vinblastine, vindesine, etoposide, podophyllotoxin, docetaxel, paclitaxel, or vinorelbine.

The antitumor platinum complexes may include cisplatin, carboplatin, nedaplatin, or oxaliplatin.

The antitumor camptothecin derivatives may include irinotecan, topotecan, or camptothecin.

The antitumor kinase inhibitors may include gefitinib, imatinib, or erlotinib.

The antitumor antibodies may include cetuximab, bevacizumab, rituximab, bevacizumab, alemtuzumab, or trastuzumab.

The hormonal antitumor agents may include goserelin, leuprolide, or tamoxifen.

The antitumor viral agents may include Imlygic (talimogene laherparepvec).

The angiogenesis inhibitors may include Avastin, bevacizumab, ranibizumab, pegaptanib, aflibercept, axitinib, cabozantinib, aflibercept, brivanib, tivozanib, ramucirumab, or motesanib, but with no limitations thereto.

Additionally, the present disclosure relates to a pharmaceutical composition for the prevention or treatment of cancer, characterized by the combined administration of a compound represented by Chemical Formulas 1, 2, 3, or 4 along with a cell therapy.

The cell therapy may be selected from lymphokine-activated killer cells (LAK), dendritic cells (DC), natural killer (NK) cells, tumor-infiltrating lymphocytes (TIL), engineered T cell receptor therapy cells (TCR-T), chimeric antigen receptor-modified T cells (CAR-T), or chimeric antigen receptor-modified NK cells (CAR-NK).

Furthermore, the present disclosure relates to a composition containing a compound represented by Chemical Formulas 1, 2, 3, or 4, or a pharmaceutically acceptable salt thereof, for the production of immune-activating cells via ex vivo culturing of therapeutic cells used in immunomodulatory cell therapies, or for the amplification of immune cell production.

Additionally, the present disclosure relates to a method for treating a subject suffering from cancer, the method comprising a step of administering an effective amount of a compound represented by Chemical Formulas 1, 2, 3, or 4, or a pharmaceutically acceptable salt thereof, and an effective amount of the immunomodulatory cell therapy or immunomodulatory agent, such as a checkpoint inhibitor (e.g., anti-PD-1 antibody, anti-CTLA4 antibody, or anti-PD-L1 antibody) or tryptophan oxidation inhibitor (e.g., IDO1, IDO2, or TDO2 inhibitor), to the subject.

The pharmaceutical composition according to the present disclosure may be formulated in an appropriate form with pharmaceutically acceptable carriers generally used in the field. The term "pharmaceutically acceptable" means pertaining to what is physiologically acceptable and cause no typical allergic reactions or similar responses, such as gastrointestinal disorders or dizziness, when administered to humans. Additionally, the composition may be formulated into oral formulations such as powders, granules, tablets, capsules, suspensions, emulsions, syrups, or aerosols; topical formulations; suppositories; or sterile injectable solutions, using conventional methods.

The carriers, excipients, and diluents that may be included in the composition include lactose, dextrose, sucrose, sorbitol, mannitol, xylitol, erythritol, maltitol, starch, arabic gum, alginate, gelatin, calcium phosphate, calcium silicate, cellulose, methylcellulose, microcrystalline cellulose, polyvinylpyrrolidone, water, methyl paraoxybenzoate, propyl paraoxybenzoate, talc, magnesium stearate, and mineral oils, but are not limited thereto. When formulating, common fillers, stabilizers, binders, disintegrants, surfactants, or other excipients may be used. Solid formulations for oral administration include tablets, pills, powders, granules, and capsules. These solid formulations are prepared by mixing at least one excipient such as starch, microcrystalline cellulose, sucrose, lactose, low-substituted hydroxypropyl cellulose, or hypromellose with the compound of the present disclosure. In addition to simple excipients, lubricants such as magnesium stearate and talc may also be used. Liquid formulations for oral administration include suspensions, solutions, emulsions, and syrups. They may contain commonly used diluents such as water or liquid paraffin, as well as excipients such as wetting agents, sweeteners, flavoring agents, and preservatives. Non-oral formulations include sterile aqueous solutions, non-aqueous solvents, suspensions, emulsions, lyophilized formulations, and suppositories. Non-aqueous solvents or suspension agents such as propylene glycol, polyethylene glycol, vegetable oils like olive oil, and injectable esters such as ethyl oleate can be used. Suppository bases may include Witepsol, macrogol, Tween 61, cacao butter, laurin butter, glycerol, and gelatin. For non-oral administration, the isobenzofuran-1(3H)-one derivatives represented by Chemical Formulas 1, 2, 3, or 4, or pharmaceutically acceptable salts thereof, may be mixed with sterile water or preservatives, stabilizers, hydrating agents, or emulsifying agents, osmoregulators, salts, or buffers, and other therapeutically useful substances, to prepare solutions or suspensions. These may be manufactured into unit dosage forms like ampoules or vials for injection.

The pharmaceutical composition comprising, as an active ingredient, the compound represented by Chemical Formulas 1, 2, 3, or 4, as disclosed in the present disclosure, may be administered to mammals such as mice, livestock, or humans through various routes.

All modes of administration may be contemplated, including, for example, oral, rectal, intravenous, intramuscular, subcutaneous, intrauterine, intradural, or intracerebrovascular injection. The dosage will vary depending on factors such as the age, sex, body weight, specific disease or pathological condition to be treated, severity of the disease or pathological condition, time of administration, route of administration, rate of absorption, distribution and excretion of the drug, the type of other drugs used, and the judgment of the prescribing physician. Determining the dosage based on these factors is within the knowledge of those skilled in the art. Typically, the dosage range is from approximately 0.01 mg/kg/day to about 2000 mg/kg/day, with a more preferred dosage range being from 1 mg/kg/day to 500 mg/kg/day. The dosage may be administered once a day or divided into several doses. The above-mentioned dosage does not limit the scope of the present disclosure in any way.

Additionally, the pharmaceutical composition of the present disclosure may be used alone or in combination with surgery, hormone therapy, chemotherapy, and biological response modifiers for the prevention or treatment of cancer, viral infections, Parkinson's disease, non-alcoholic steatohepatitis, or tuberculosis.

### Advantageous Effects of Invention

The present disclosure relates to isobenzofuran-1(3H)-one derivatives and uses thereof. The isobenzofuran-1(3H)-one derivatives have HPK1 inhibitory activity and MLK3 inhibitory activity and are useful as pharmaceutical compositions for the prevention or treatment of cancer, viral infections, Parkinson's disease, non-alcoholic steatohepatitis, or tuberculosis. Moreover, the isobenzofuran-1(3H)-one derivatives of the present disclosure exhibit excellent anticancer effects when administered in combination with anticancer drugs or cell therapies, making them valuable pharmaceutical compositions for cancer prevention or treatment.

### Brief Description of Drawings

FIG. 1 shows the anticancer activity of the compound of the present disclosure against the human ovarian cancer cell line A2780 and the cisplatin-resistant cell line A2780/cis.
FIG. 2 shows the enhancing effect of the compound of the present disclosure on T cell activity.

### Best Mode for Carrying out the Invention

Hereinafter, preferable embodiments of the present disclosure will be described in detail. However, the present disclosure is not limited to the embodiments described herein but may be embodied in other forms. Rather, these embodiments are provided so that this disclosure will be thorough and complete, and will fully convey the scope of the invention to those skilled in the art.

### <EXAMPLE 1. Synthesis of Isobenzofuran-1(3H)-on Derivatives and Physicochemical Characterization>

Synthesis procedures and physicochemical properties of compounds 1 to 3 of the present disclosure are as follows.

### Compound 1. 5-((5-chloro-2-((1,2,3,4-tetrahydroisoquinolin-6-yl)amino)pyrimidin-4-yl)amino)-3,3-dimethylisobenzofuran-1(3H)-one

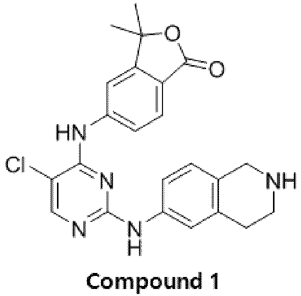

### Step 1: Synthesis of 2-(5-bromo-2-(hydroxymethyl)phenyl)propan-2-ol

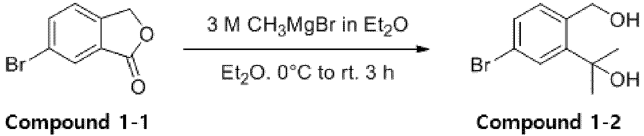

A solution of compound 1-1 (6-bromodimethylisobenzofuran-1(3H)-one; 1.00 g, 4.69 mmol) in Et₂O(30 ml) was filled with nitrogen for 5 minutes, followed by slowly adding drops of 3 M CH₃MgBr in Et₂O(4.69 ml, 14.1 mmol) at 0°C for 20 minutes. The mixture was left at room temperature for 3 hours. Thereafter, the reaction mixture was cooled to 0°C and quenched with saturated NH₄Cl (10 mL). The crude mixture was subjected to extraction with MC and the extract was washed with brine and dried over MgSO₄ to afford compound 1-2 as a colorless oil (1.15 g, 4.69 mmol).

### Step 2: Synthesis of 5-bromo-3,3-dimethylisobenzofuran-1(3H)-one

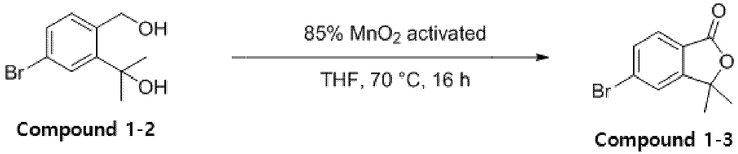

A solution of compound 1-2(1.15 g, 4.69 mmol) in THF(10 mL) was added with 85% activated MnO₂(4.08 g, 46.9 mmol) and then stirred at 70°C for 16 hours. After 7 hours, the reaction mixture was filtered through celite and the filtrate was concentrated and softened with EA and Hex to afford compound 1-3 as a white solid (926 mg, 3.84 mmol).

### Step 3: Synthesis of tert-butyl (3,3-dimethyl-1-oxo-1,3-dihydroisobenzofuran-5-yl)carbamate

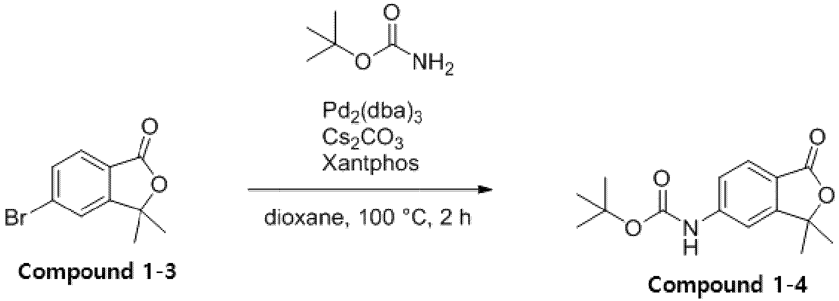

Pd₂(dba)₃(57.0 mg, 0.0620 mmol) and Xantphos(36.0 mg, 0.0207 mmol) were introduced into an oven-dried tube, placed under a vacuum, and then filled with nitrogen. Anhydrous 1,4-dioxane (2 mL), compound 1-3 (500 mg, 2.07 mmol), and tert-butyl carbamate (364 mg, 3.11 mmol) were added to the reaction tube. After addition of Cs₂CO₃(1.35 g, 4.15 mmol), the tube was sealed and the mixture was stirred at 100°C for 2 hours. After completion of the reaction, the reaction mixture was quenched with water and subjected to extraction with DCM. The extract was concentrated in a vacuum. The crude product was purified on silica gel by column chromatography (eluent: 30% EA in Hex) to afford compound 1-4 as a white solid (250 mg, 0.901 mmol, 44%).

### Step 4: Synthesis of 5-amino-3,3-dimethylisobenzofuran-1(3 H )-one

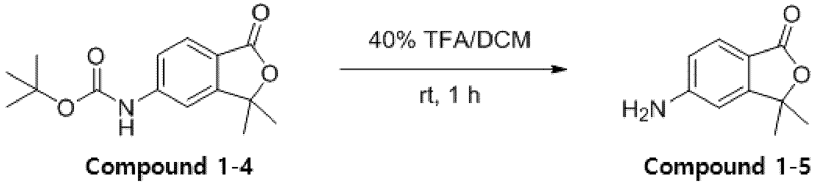

A solution of compound 1-4 (250 mg, 0.901 mmol) in DCM (1.5 mL) was added with 40% TFA and then stirred for 1 hour. The reaction mixture was quenched with water, alkalinized with saturated NaHCO₃(aq.), and subjected to extraction with DCM (30 ml x 2). The crude mixture was purified using MPLC (eluent: 15% EA in Hex) to afford compound 1-5 as a white solid (130 mg, 0.901 mmol, 81%).

### Step 5: Synthesis of 5-((2,5-dichloropyrimidin-4-yl)amino)-3,3-dimethylisobenzofuran-1(3H)-one

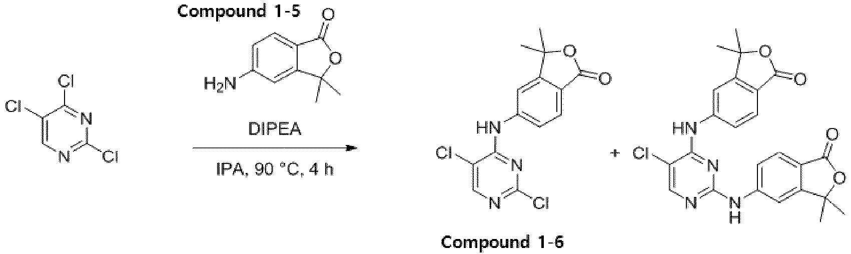

A solution of 2,4,5-trichloropyrimidine (100 mg, 0.545 mmol) in IPA(1.5 mL) was added with DIPEA(114 uL, 0.654 mmol) and then stirred. To the mixture was added compound 1-5 (116 mg, 0.654 mmol) before stirring at 90°C for 4 hours. The reaction mixture was cooled, dissolved in EA, and washed with water. The organic layer was dried over MgSO₄ and concentrated using a rotary evaporator. The concentrate was purified by MPLC (20% EA/Hex) to afford compound 1-6 as a white solid (46.0 mg, 0.142 mmol, 26%).

### Step 6: Synthesis of 5-((5-chloro-2-((2-(2,2,2-trifluoroacetyl)-1,2,3,4-tetrahydroisoquinolin-6-yl)amino)pyrimidin-4-yl)amino)-3,3-dimethylisobenzofuran-1(3 H )-one

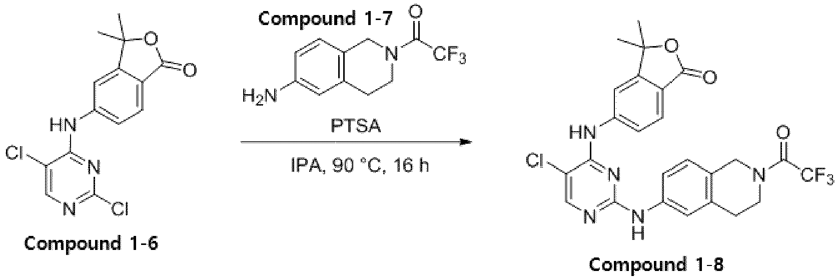

A solution of compound 1-6 (43.0 mg, 0.133 mmol) in IPA (1 mL) was added with compound 1-7 (35.6 mg, 0.146 mmol) and PTSA (22.8 mg, 0.123 mmol) and then stirred at 90°C for 16 hours. The reaction mixture was quenched with water and subjected to extraction with EA(30 mL x 2). The organic layer was dried over MgSO₄ and concentrated in a vacuum. The concentrate was purified by MPLC (30% EA/Hex) to afford 1-8 as a white solid (34.0 mg, 0.0064 mmol, 48%).

### Step 7: 5-((5-chloro-2-((1,2,3,4-tetrahydroisoquinolin-6-yl)amino)pyrimidin-4-yl)amino)-3,3-dimethylisobenzofuran-1(3H)-one (compound 1)

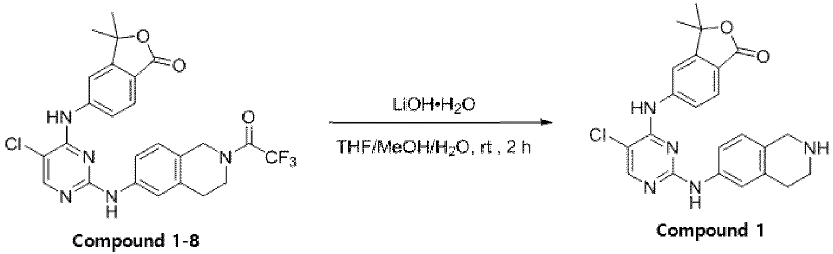

A solution of compound 1-8 (30.0 mg, 0.0560 mmol) in THF/MeOH/H₂O (1 mL) was added with LiOH·H₂O (2.37 mg, 0.0560 mmol) and then stirred for 2 hours. The reaction mixture was purified using 1 N HCl, NaHCO₃ (acid/base post-treatment) to afford compound 1 as a white solid (19.0 mg, 0.0440 mmol).

¹H NMR (400 MHz, DMSO-*d*₆) δ 9.27 (s, 1H), 9.19 (s, 1H), 8.23 (s, 1H), 7.98 (dd, *J*= 8.4, 1.8 Hz, 1H), 7.92 (s, 1H), 7.71 (d, *J*= 8.4 Hz, 1H), 7.30 (dd, *J*= 8.3, 2.2 Hz, 1H), 7.25 (s, 1H), 6.86 (d, *J*= 8.3 Hz, 1H), 3.76 (s, 2H), 2.88 (t, *J*= 5.8 Hz, 2H), 2.53-2.51 (m, 2H), 1.57 (s, 6H).
LC/MS : 436.39 [M+H]⁺

### Compound 2. 6-((5-chloro-2-((1,2,3,4-tetrahydroisoquinolin-6-yl)amino)pyrimidin-4-yl)amino)-3,3-dimethylisobenzofuran-1(3H)-one

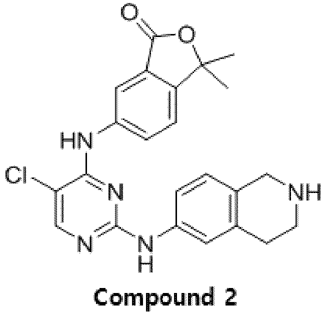

### Step 1: Synthesis of 3,3-dimethylisobenzofuran-1(3 H )-one

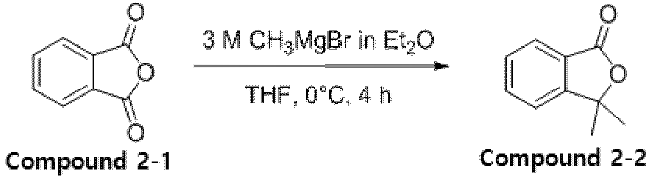

A solution of compound 2-1 (isobenzofuran-1,3-dione; 1.00 g, 6.75 mmol) in THF (30 mL) in a one-neck round-bottom flask was stirred under a nitrogen atmosphere and added with drops of MeMgBr (3 M, 4.97 mL, 14.9 mmol) in Et₂O at 0°C within an ice bath. The resulting solution was stirred at room temperature for 4 hours. The reaction was quenched by adding 150 mL of HCl (10 wt%). The reaction mixture was subjected to extraction with EtOAc (100 mL x 2), and the pooled organic phase was dried over anhydrous sodium sulfate. The solid was filtered and the filtrate was concentrated in a vacuum. The residue was eluted from silica gel using EtOAc/Hex(1:9) to afford compound 2-2 as a white solid (439 mg, 2.71 mmol).

### Step 2: Synthesis of 3,3-dimethyl-6-nitroisobenzofuran-1(3 H )-one

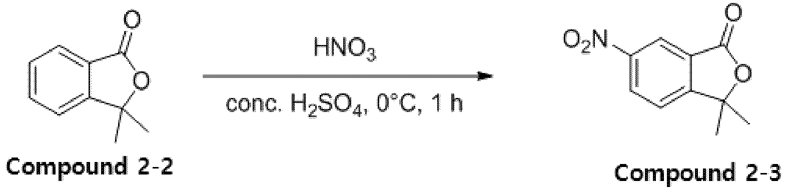

A solid of compound 2-2 (3,3-dimethyldimethylisobenzofuran-1(3H)-one; 33.0 mg, 0.206 mmol) in H₂SO₄(1 mL) was added with nitric acid (19.2 mg, 0.305 mmol) and stirred at 0°C for 1 hour. The reaction was quenched by adding water to the reaction mixture, followed by extraction with EA (20 mL x 2). The organic layer was dried over MgSO₄ and concentrated using a rotary evaporator to afford compound 2-3 as a white solid (41.2 mg, 0.203 mmol).

### Step 3: Synthesis of 6-amino-3,3-dimethylisobenzofuran-1(3H)-one

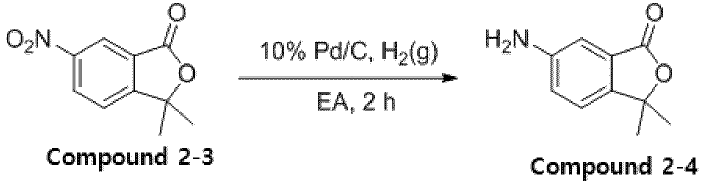

A solution of compound 2-3 (3,3-dimethyl-5-nitroisobenzofuran-1(3H)-one; 33.0 mg, 0.159 mmol) in EA(5 mL) was added with 10% Pd/C (5 mg) and stirred for 2 hours. The reaction mixture was filtered through a celite filter and concentrated using an evaporator to afford compound 2-4 as a white solid (28.2 mg, 0.159 mmol) .

### Step 4: Synthesis of 6-((2,5-dichloropyrimidin-4-yl)amino)-3,3-dimethylisobenzofuran-1(3 H )-one

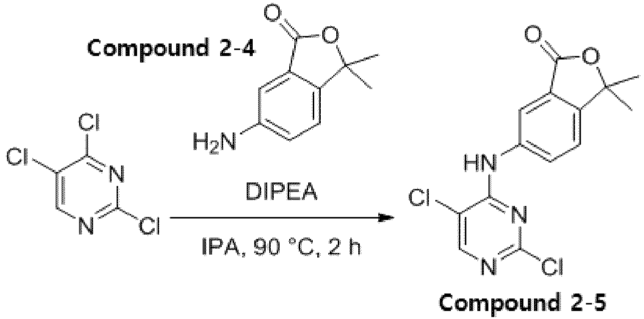

A solution of 2,4,5-trichloropyrimidine (20.0 mg, 0.109 mmol) in IPA(1.5 mL) was added with DIPEA (23.0 uL, 0.131 mmol) and then stirred. To this mixture was added compound 2-4 (6-amino-3,3-dimethyldimethylisobenzofuran-1(3*H*)-one; 20.3 mg, 0.114 mmol), followed by stirring at 90°C for 2 hours. The reaction mixture was cooled, dissolved in EA, and washed with water. The organic layer was dried over MgSO₄, and concentrated using a rotary evaporator. The concentrate was purified by MPLC (20% EA/Hex) to afford compound 2-5 as a white solid (33.9 mg, 0.109 mmol).

### Step 5: Synthesis of 6-((5-chloro-2-((2-(2,2,2-trifluoroacetyl)-1,2,3,4-tetrahydroisoquinolin-6-yl)amino)pyrimidin-4-yl)amino)-3,3-dimethylisobenzofuran-1(3H)-one

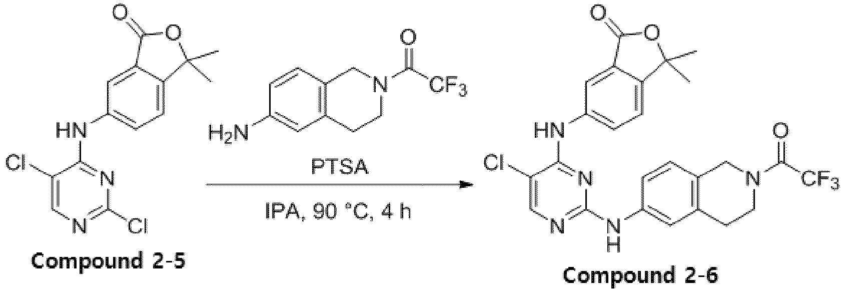

A solution of compound 2-5(20.0 mg, 0.0620 mmol) in IPA (1 mL) was added with 1-(6-amino-3,4-dihydroisoquinolin-2(1H)-yl)-2,2,2-trifluoroethan-1-one (16.6 mg, 0.0680 mmol) and PTSA(10.6 mg, 0.0620 mmol) and then stirred 90°C for 4 hours. The reaction mixture was quenched with water and then subjected to extraction with EA (30 mL x 2). The organic layer was dried over MgSO₄ and concentrated in a vacuum. The concentrate was purified by MPLC (30% EA/Hex) to afford compound 2-6 as a white solid (20.6 mg, 0.0390 mmol, 63%).

### Step 6: Synthesis of 6-((5-chloro-2-((1,2,3,4-tetrahydroisoquinolin-6-yl)amino)pyrimidin-4-yl)amino)-3,3-dimethylisobenzofuran-1(3H)-one (compound 2)

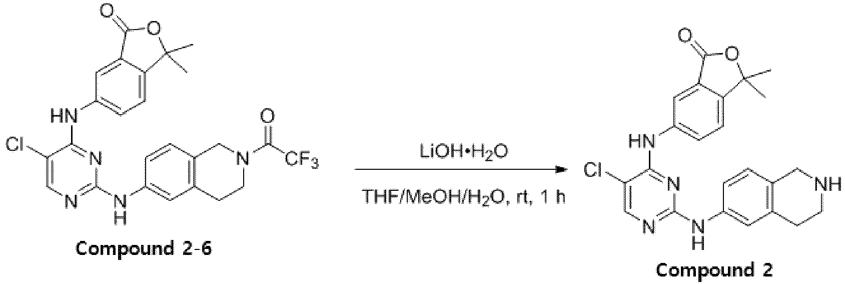

A solution of compound 2-6(20.0 mg, 0.038 mmol) in THF/MeOH/H₂O(1 mL) was added with LiOH·H₂O(4.74 mg, 0.113 mmol) and then stirred at room temperature for 1 hour. Reaction with acid/base afforded compound 2 as a white solid (9.5 mg, 0.0220 mmol).

¹H NMR (400 MHz, DMSO- *d* ₆) δ 9.26 (s, 1H), 9.13 (s, 1H), 8.18 (s, 1H), 8.05 (dd, *J*= 8.2, 2.0 Hz, 1H), 7.91 (d, *J*= 2.0 Hz, 1H), 7.70 (d, *J*= 8.2 Hz, 1H), 7.36 (d, *J*= 2.2 Hz, 1H), 7.28-7.13 (m, 1H), 6.80 (d, *J*= 8.3 Hz, 1H), 3.74 (s, 2H), 3.51 (s, 1H), 2.86 (t, *J*= 5.9 Hz, 2H), 2.45-2.40 (m, 2H), 1.65 (s, 6H).
LC/MS: 434.43 [M-H] ⁺

### Compound 3. 5,5'-((5-Chloropyrimidine-2,4-diyl)bis(azanediyl))bis(3,3-dimethylisobenzofuran-1(3H)-one)

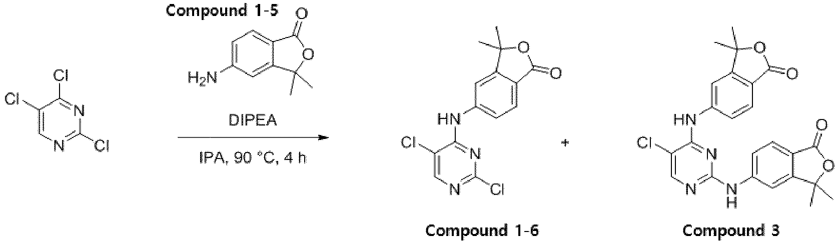

A solution of 2,4,5-trichloropyrimidine (100 mg, 0.545 mmol) in IPA(1.5 mL) was added with DIPEA (114 uL, 0.654 mmol) and then stirred. To this mixture was added compound 1-5 (116 mg, 0.654 mmol) before stirring at 90°C for 4 hours. The reaction mixture was cooled, dissolved in EA, and washed with water. The organic layer was dried over MgSO₄, and concentrated using a rotary evaporator. The reaction mixture was purified by MPLC (20% EA/Hex) to afford compound 3 as a white solid (34.0mg, 0.073 mmol, 13%).

¹H NMR (400 MHz, CDCl₃) δ 8.28 (s, 1H), 7.97-7.88 (m, 2H), 7.83-7.75 (m, 2H), 7.61 (dd, *J*= 8.4, 1.8 Hz, 1H), 7.54 (d, *J*= 1.7 Hz, 1H), 7.42 (s, 1H), 7.32 (s, 1H), 1.70 (s, 6H), 1.66 (s, 6H).

### EXPERIMENTAL EXAMPLE 1. Evaluation for Inhibition of Inventive Compounds against Kinase Activity of MLK3 or HPK1

### 1-1. Evaluation for Inhibition against Kinase Activity of MLK3

The kinase inhibition activity against MLK3 was measured as follows. Recombinant human MKK3 protein was mixed with the compounds in a reaction solution (8 mM MOPS, pH 7.0, 0.2 mM EDTA, 0.33 mg/mL myelin basic protein, 5 mM DTT, 10 mM magnesium acetate, and 45 µM [gamma-33P-ATP]). The mixture was incubated at room temperature for 40 minutes. The reaction was terminated by adding phosphoric acid at a final concentration of 0.5%. A 10 µL aliquot of the reaction solution was then spotted onto a P30 filter, which was washed four times in 0.425% phosphoric acid solution for 4 minutes each and then washed once in methanol. The filters were dried, and MLK3 activity was measured via scintillation counting. The MLK3 activity observed in the control group free of the compounds was set as 100%, and the inhibition by the compounds was expressed relative to this control value. To calculate the IC₅₀ value, the compounds were diluted in 9 concentrations, starting from a maximum of 1 µM in a threefold serial dilution. The IC₅₀ was defined as the concentration at which 50% inhibition was observed.

### 1-2. Evaluation for Inhibition against Kinase Activity of HPK1

Inhibition against kinase activity of HPK1 was measured as follows. Recombinant human HPK1 protein was mixed with the compounds in a reaction solution (8 mM MOPS, pH 7.0, 0.2 mM EDTA, 0.33 mg/mL myelin basic protein, 10 mM magnesium acetate, and 15 µM [gamma-33P-ATP]). The mixture was incubated at room temperature for 40 minutes. The reaction was terminated by adding phosphoric acid at a final concentration of 0.5%. A 10 µL aliquot of the reaction solution was spotted onto a P30 filter, washed four times in 0.425% phosphoric acid solution for 4 minutes each, and washed once with methanol. The filters were dried, and HPK1 activity was measured via scintillation counting. The HPK1 activity observed in the control group free of the compounds was set as 100%, and the inhibition by the compounds was expressed relative to this control value. To calculate the IC₅₀ value, the compounds were diluted in 9 concentrations, starting from a maximum of 1 µM in a threefold serial dilution. The IC₅₀ was defined as the concentration at which 50% inhibition was observed.

### 1-3. Results of Inhibition against Kinase Activity of MLK3 or HPK1

The inhibition against kinase activity of MLK3 or HPK1 was evaluated using compounds 1 to 3 synthesized in Example 1. The results are given in Table 1.

**TABLE 1**

| **Kinase** | **Compound** | **IC₅₀ (nM)** |
|---|---|---|
| HPK1 (h) | Compound 1 | ++++ |
| | Compound 2 | ++++ |
| | Compound 3 | ++ |
| MLK3 (h) | Compound 1 | +++ |
| | Compound 2 | +++ |
| | Compound 3 | + |

| | | |
|---|---|---|
| +: IC₅₀ > 1000 nM ++: 500 < IC₅₀ < 1000 nM +++: 100 < IC₅₀ < 500 nM ++++: 1 < IC₅₀ < 100 nM IC₅₀ values | | |

As shown in Table 1, the IC₅₀ value for HPK1 inhibition by compound 1 was approximately 30 nM, by compound 2 approximately 50 nM, and by compound 3 approximately 900 nM. The IC₅₀ value for MLK3 inhibition by compound 1 was approximately 140 nM, and by compound 2 approximately 200 nM. The isobenzofuran-1(3H)-one derivatives of the present disclosure demonstrated potent dual inhibition of HPK1 and MLK3 at low concentrations (nM).

### <EXPERIMENTAL EXAMPLE 2. Anticancer Efficacy of Inventive Compounds>

The anticancer activity of the MLK3 and HPK1 kinase inhibitor compounds was evaluated using the human ovarian cancer cell line A2780 and the cisplatin-resistant cell line A2780/cis. A2780 and A2780/cis cells were cultured at a density of 4000 cells per well in 96-well plates and stabilized for one day. The compounds 1 to 3 synthesized in Example 1 were serially diluted starting from a concentration of 10 µM and applied to the cells. The treated cells were incubated for three days, and cell viability was measured using CCK8 reagent, with the concentration at which 50% of the cancer cells died expressed as the GI₅₀.

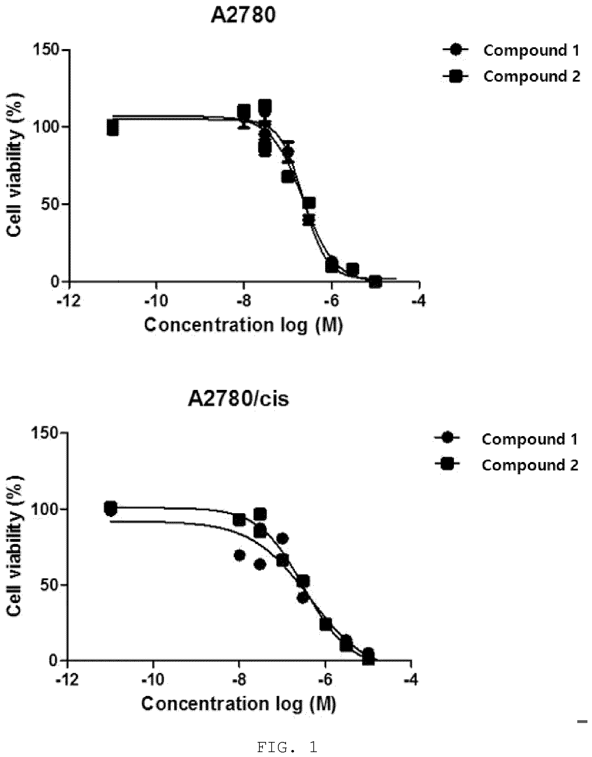

Referring to FIG. 1, the concentration needed to inhibit the growth of A2780 cells by 50% (GI₅₀) was approximately 200 nM for compound 1 and 210 nM for compound 2. With regard to A2780/cis cells, GI₅₀ was approximately 400 nM for compound 1 and 310 nM for compound 2. These results confirm that the isobenzofuran-1(3H)-one derivatives (compounds 1 to 3) of the present disclosure exhibit anticancer efficacy in both the ovarian cancer cell line A2780 and the cisplatin-resistant cell line A2780/cis.

Additionally, the anticancer activity of the compounds was also observed in other cancer cell lines (blood cancer, breast cancer, ovarian cancer, lung cancer, and brain cancer), as well as in drug-resistant cancer cell lines (doxorubicin-resistant blood cancer K562/ADM; and paclitaxel-resistant breast cancer MDA-MB-231/PTX). The anticancer activity results of the compounds are shown in Table 2.

**TABLE 2**

| GI₅₀ (µM) | Compound 1 | Compound 2 | Compound 3 |
|---|---|---|---|
| K562 | 0.43 | 1.07 | >10 µM |
| K562/ADM | 3.74 | 2.28 | >10 µM |
| U937 | 0.29 | 0.44 | >10 µM |
| MDA-MB-231 | 1.25 | 1.03 | 1.35 |
| MDA-MB-231/PTX | 0.99 | 0.77 | 1.11 |
| MDA-MB-453 | 1.02 | 2.90 | 1.07 |
| SKOV3 | 1.704 | 1.50 | >10 µM |
| A549 | 1.61 | 1.15 | >10 µM |
| SH-SY5Y | 0.39 | 0.28 | 0.55 |

Referring to Table 2, it is confirmed that compounds 1, 2, and 3 exhibit anticancer activity across various cancer cell lines, including drug-resistant cancer cell lines.

### <EXPERIMENTAL EXAMPLE 3. Measurement of IL-2 for Evaluation of T Cell Activation Enhancement by Inventive Compounds>

The ability to enhance T cell activation through HPK1 inhibition was observed in the following experiment. Human PBMC was seeded at a density of 2 x 10⁵ cells per well in a 96-well plate. The cells were pretreated for one hour with serial dilutions of the compounds 1 to 3 synthesized in Example 1 starting from 3 µM. Afterward, CD3/CD28 Dynabeads were added to the cells at a 1:1 ratio and incubated for 6 days. After incubation, the cells and media were analyzed using the Sartorius human T cell activation cell and cytokine profiling kit with the iQue system. IL-2, a biomarker used to assess T cell activation, was measured. The results were calculated by setting the positive control group (treated with Dynabeads alone) as 100%.

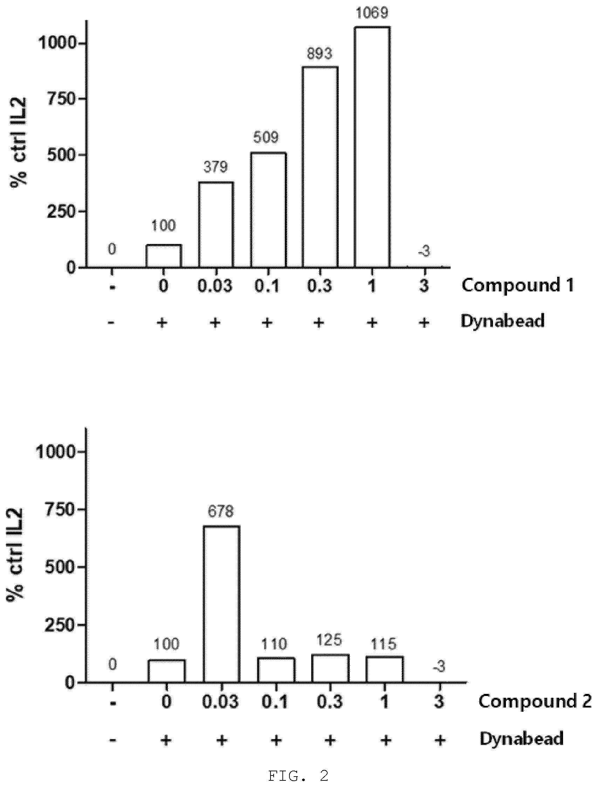

Referring to FIG. 2, it was observed that compounds 1 and 2 increased IL-2 levels, indicating that these compounds possess T cell activation-enhancing activity.

Furthermore, the characteristic HPK1 and MLK3 inhibitory activity of the isobenzofuran-1(3H)-one derivatives of the present disclosure was confirmed to have a synergistic anticancer effect. This was achieved through combination therapy with conventional anticancer drugs or by co-administering the compounds during ex vivo culture of therapeutic cells used in immunomodulatory cell therapy, leading to the production of immune-enhanced cells or the proliferation of immune cells.

### <FORMULATION EXAMPLE 1. Preparation of Tablets>

A mixture of 100 mg of Compound 1 of the present disclosure, 100 mg of microcrystalline cellulose, 60 mg of lactose monohydrate, 20 mg of low-substituted hydroxypropyl cellulose, and 2 mg of magnesium stearate was compressed into tablets using a conventional tablet preparation method.

### <FORMULATION EXAMPLE 2. Preparation of Capsules>

A mixture of 100 mg of Compound 1 of the present disclosure, 100 mg of microcrystalline cellulose, 60 mg of lactose monohydrate, 20 mg of low-substituted hydroxypropyl cellulose, and 2 mg of magnesium stearate was loaded into gelatin capsules using a standard capsule preparation method.

### <FORMULATION EXAMPLE 3. Preparation of Injectable Solution>

A mixture of 10 mg of Compound 1 of the present disclosure, a suitable amount of sterile distilled water for injection, and a suitable amount of pH adjuster was prepared into an injectable solution per ampoule (2 mL) using a conventional method for manufacturing injectable solutions.

## Claims

1. A compound represented by Chemical Formula 1 or 2, an optical isomer thereof, or a pharmaceutically acceptable salt thereof: 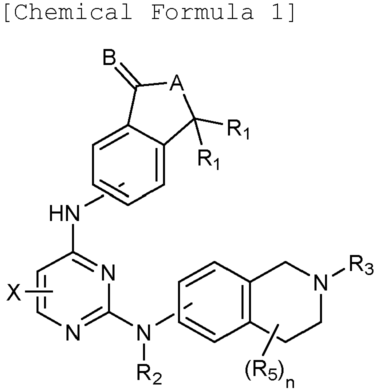 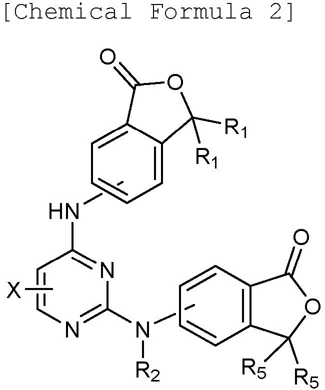 wherein,
R₁'s are each independently a hydrogen atom, a C₁-C₄ alkyl, or a C₁-C₄ alkoxy,
or the two R₁'s form a 3- to 7-membered saturated spiro ring with an adjacent carbon atom (C);
R₂ is a hydrogen atom, a C₁-C₄ alkyl, or a C₁-C₄ alkoxy;
R₃ is a hydrogen atom, a C₁-C₄ alkyl, a halo C₁-C₄ alkyl, a hydroxy C₁-C₄ alkyl, a C₁-C₄ alkoxy, a halo C₁-C₄ alkoxy, a C₁-C₄ alkoxy C₁-C₄ alkoxy, -COR₄, -CONH₂, - CONHR₄, -CON(R₄)₂, -SO₃H, or -SO₂R₄;
R₄ is a substituted or unsubstituted trigonal to dodecagonal cycloalkyl, a substituted or unsubstituted trigonal to dodecagonal heterocycloalkyl, a substituted or unsubstituted tetragonal to dodecagonal aryl, a substituted or unsubstituted tetragonal to dodecagonal heteroaryl, a substituted or unsubstituted trigonal to dodecagonal cycloalkyl C₁-C₄ alkyl, a substituted or unsubstituted trigonal to dodecagonal heterocycloalkyl C₁-C₄ alkyl, a substituted or unsubstituted tetragonal to dodecagonal aryl C₁-C₄ alkyl, a substituted or unsubstituted tetragonal to dodecagonal heteroaryl C₁-C₄ alkyl, a substituted or unsubstituted C₁-C₄ alkyl, a halo C₁-C₄ alkyl, a hydroxy C₁-C₄ alkyl, a C₁-C₄ alkoxy, a halo C₁-C₄ alkoxy, or a C₁-C₄ alkoxy C₁-C₄ alkoxy,
wherein the substituted cycloalkyl, heterocycloalkyl, aryl, heteroaryl, cycloalkylalkyl, heterocycloalkylalkyl, arylalkyl, heteroarylalkyl, or alkyl has at least one substituent selected from the group consisting of OH, NO₂, NH₂, CN, a halogen, a C₁-C₄ alkyl, a halo C₁-C₄ alkyl, a hydroxy C₁-C₄ alkyl, a C₁-C₄ alkoxy, a halo C₁-C₄ alkoxy, and a C₁-C₄ alkoxy C₁-C₄ alkoxy, at a position to which a hydrogen atom can be bonded;
R₅'s are each independently a hydrogen atom, a C₁-C₄ alkyl, or a C₁-C₄ alkoxy,
or the two R₅'s form a 3- to 7-membered saturated spiro ring with an adjacent carbon atom (C);
A is O or NH;
B is O or S;
X is a halogen atom; and
n is an integer of 0 to 6.

2. The compound, optical isomer, or pharmaceutically acceptable salt of claim 1, wherein the compound is represented by the following Chemical Formula 3 or 4: 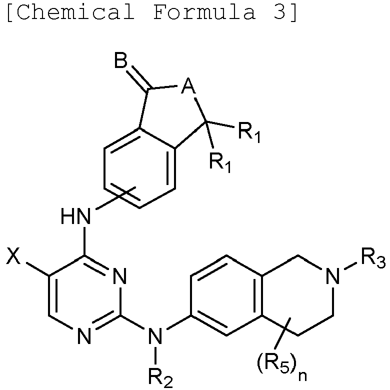 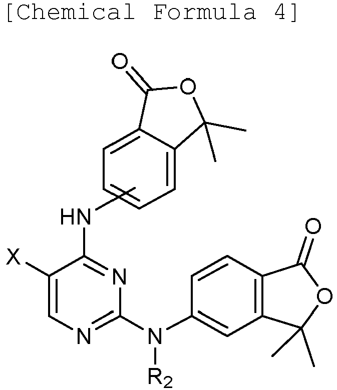 wherein,
R₁'s are each independently a hydrogen atom, a C₁-C₄ alkyl, or a C₁-C₄ alkoxy,
or the two R₁'s form a 3- to 7-membered saturated spiro ring with an adjacent carbon atom (C);
R₂ is a hydrogen atom, a C₁-C₄ alkyl, or a C₁-C₄ alkoxy;
R₃ is a hydrogen atom, a C₁-C₄ alkyl, a halo C₁-C₄ alkyl, a hydroxy C₁-C₄ alkyl, a C₁-C₄ alkoxy, a halo C₁-C₄ alkoxy, a C₁-C₄ alkoxy C₁-C₄ alkoxy, -COR₄, -CONH₂, - CONHR₄, -CON(R₄)₂, -SO₃H, or -SO₂R₄;
R₄ is a substituted or unsubstituted trigonal to dodecagonal cycloalkyl, a substituted or unsubstituted trigonal to dodecagonal heterocycloalkyl, a substituted or unsubstituted tetragonal to dodecagonal aryl, a substituted or unsubstituted tetragonal to dodecagonal heteroaryl, a substituted or unsubstituted trigonal to dodecagonal cycloalkyl C₁-C₄ alkyl, a substituted or unsubstituted trigonal to dodecagonal heterocycloalkyl C₁-C₄ alkyl, a substituted or unsubstituted tetragonal to dodecagonal aryl C₁-C₄ alkyl, a substituted or unsubstituted tetragonal to dodecagonal heteroaryl C₁-C₄ alkyl, a substituted or unsubstituted C₁-C₄ alkyl, a halo C₁-C₄ alkyl, a hydroxy C₁-C₄ alkyl, a C₁-C₄ alkoxy, a halo C₁-C₄ alkoxy, or a C₁-C₄ alkoxy C₁-C₄ alkoxy,
wherein the substituted cycloalkyl, heterocycloalkyl, aryl, heteroaryl, cycloalkylalkyl, heterocycloalkylalkyl, arylalkyl, heteroarylalkyl or alkyl has at least one substituent selected from the group consisting of OH, NO₂, NH₂, CN, halogen, a C₁-C₄ alkyl, a halo C₁-C₄ alkyl, a hydroxy C₁-C₄ alkyl, a C₁-C₄ alkoxy, a halo C₁-C₄ alkoxy, and a C₁-C₄ alkoxy C₁-C₄ alkoxy, at a position to which a hydrogen atom can be bonded;
R₅'s are each independently a hydrogen atom, a C₁-C₄ alkyl, or a C₁-C₄ alkoxy,
or the two R₅'s form a 3- to 7-membered saturated spiro ring with an adjacent carbon atom (C);
A is O or NH;
B is O or S;
X is a halogen atom; and
n is an integer of 0 to 6.

3. The compound, optical isomer, or pharmaceutically acceptable salt of claim 1 or 2, wherein the compound represented by Chemical Formula 1, 2, 3, or 4 is selected from the group consisting of:
5-((5-chloro-2-((1,2,3,4-tetrahydroisoquinolin-6-yl)amino)pyrimidin-4-yl)amino)-3,3-dimethylisobenzofuran-1(3H)-one (compound 1);
6-((5-chloro-2-((1,2,3,4-tetrahydroisoquinolin-6-yl)amino)pyrimidin-4-yl)amino)-3,3-dimethylisobenzofuran-1(3H)-one (compound 2); and
5,5'-((5-chloropyrimidine-2,4-diyl)bis(azanediyl))bis(3,3-dimethylisobenzofuran-1(3H)-one) (compound 3);

4. A pharmaceutical composition for prevention or treatment of cancer, viral infectious diseases, Parkinson's disease, non-alcoholic steatohepatitis, or tuberculosis, the composition comprising as an active ingredient the compound represented by Chemical Formula 1, 2, 3, or 4, an optical isomer thereof, or a pharmaceutically acceptable salt thereof according to claim 1 or 2.

5. The pharmaceutical composition of claim 4, wherein the cancer is selected from the group consisting of lung cancer, liver cancer, stomach cancer, colorectal cancer, bladder cancer, prostate cancer, breast cancer, ovarian cancer, cervical cancer, thyroid cancer, melanoma, hematologic cancers, colon cancer, non-small cell lung cancer, pancreatic cancer, skin cancer, head and neck cancer, small intestine cancer, rectal cancer, endometrial cancer, vaginal cancer, testicular cancer, esophageal cancer, bile duct cancer, lymphomas, gallbladder cancer, endocrine gland cancer, adrenal cancer, lymphomas, multiple myeloma, thymomas, mesothelioma, kidney cancer, brain cancer, central nervous system tumors, brainstem gliomas, and pituitary adenomas.

6. The pharmaceutical composition of claim 4, wherein the viral infectious disease is caused by a virus selected from the group consisting of Zika virus, human immunodeficiency virus (HIV), influenza virus, Influenza A virus subtype H1N1, avian influenza virus, rhinovirus, adenovirus, coronavirus, parainfluenza virus, respiratory syncytial virus, herpesvirus (HSV), rotavirus, and hepatitis viruses.

7. A method for inhibiting kinase activity in a subject in need of inhibiting activity of kinases including HPK1, the method comprising administering to the subject an effective amount of the compound represented by Chemical Formula 1, 2, 3, or 4 or a pharmaceutically acceptable salt or stereoisomer thereof according to claim 1 or 2.

8. A pharmaceutical composition for prevention or treatment of cancer, the composition comprising a compound represented by Chemical Formula 1, 2, 3, or 4 according to claim 1 or 2, and an anticancer drug in combination therapy.

9. The pharmaceutical composition of claim 8, wherein the anticancer drug is selected from the group consisting of taxane-based anticancer drugs, antitumor alkylating agents, antitumor antimetabolites, antitumor antibiotics, plant-derived antitumor agents, antitumor platinum complexes, antitumor camptothecin derivatives, antitumor kinase inhibitors, antitumor antibodies, hormonal antitumor agents, antitumor viral agents, and angiogenesis inhibitors.

10. A pharmaceutical composition for prevention or treatment of cancer, the composition comprising a compound represented by Chemical Formula 1, 2, 3, or 4 according to claim 1 or 2, and a cell therapy product in combination therapy.

11. The pharmaceutical composition of claim 10, wherein the cell therapy product is selected from lymphokine-activated killer cells (LAK), dendritic cells (DC), natural killer (NK) cells, tumor-infiltrating lymphocytes (TIL), engineered T cell receptor therapy cells (TCR-T), chimeric antigen receptor-modified T cells (CAR-T), and chimeric antigen receptor-modified NK cells (CAR-NK).

12. A composition for production of immune-activating cells via ex vivo culturing of therapeutic cells used in immunomodulatory cell therapies, or for amplification of immune cell production, the composition comprising the compound represented by Chemical Formulas 1, 2, 3, or 4, or the pharmaceutically acceptable salt thereof according to claim 1 or 2.

13. A method for treating a subject suffering from cancer, the method comprising a step of administering to the subject an effective amount of the compound represented by Chemical Formulas 1, 2, 3, or 4, or a pharmaceutically acceptable salt thereof according to claim 1 or 2, and an effective amount of the immunomodulatory cell therapy or immunomodulatory agent including a checkpoint inhibitor (e.g., anti-PD-1 antibody, anti-CTLA4 antibody, or anti-PD-L1 antibody) or a tryptophan oxidation inhibitor (e.g., IDO1, IDO2, or TDO2 inhibitor) according to claim 12.
